(19) Europäisches Patentamt
European Patent Office
Office européen des brevets

(11) **EP 4 062 850 A1**

(12) **EUROPEAN PATENT APPLICATION**
published in accordance with Art. 153(4) EPC

(43) Date of publication:
**28.09.2022 Bulletin 2022/39**

(21) Application number: **20889466.7**

(22) Date of filing: **18.11.2020**

(51) International Patent Classification (IPC):
**A61B 18/20** (2006.01)   **A61N 5/067** (2006.01)

(52) Cooperative Patent Classification (CPC):
**A61B 18/20; A61N 5/06**

(86) International application number:
**PCT/JP2020/043058**

(87) International publication number:
**WO 2021/100778 (27.05.2021 Gazette 2021/21)**

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**
Designated Extension States:
**BA ME**
Designated Validation States:
**KH MA MD TN**

(30) Priority: **18.11.2019 JP 2019207610**

(71) Applicant: **FURUKAWA ELECTRIC CO., LTD.
Chiyoda-ku
Tokyo 100-8322 (JP)**

(72) Inventors:
• **OGAWA, Emiyu
  Sagamihara-shi, Kanagawa 252-0373 (JP)**

• **ARAI, Tsunenori
  Kawasaki-shi, Kanagawa 211-0011 (JP)**
• **MATSUSHITA, Shunichi
  Tokyo 100-8322 (JP)**
• **NARA, Kazutaka
  Tokyo 100-8322 (JP)**
• **YAMAUCHI, Kyosuke
  Tokyo 100-8322 (JP)**

(74) Representative: **SSM Sandmair
Patentanwälte Rechtsanwalt
Partnerschaft mbB
Joseph-Wild-Straße 20
81829 München (DE)**

(54) **PULSE IRRADIATION METHOD AND PULSE IRRADIATION DEVICE**

(57)   A pulse application method is, for example, a pulse application method of applying a pulse of light to living tissue to heat the living tissue, wherein a wavelength of the light is set within a range in which a temperature rise width of collagen fibers in the living tissue when the light is applied to the living tissue is larger than a temperature rise width of water containing cells that are contained in the living tissue and that are present around the collagen fibers.

**FIG.4**

EP 4 062 850 A1

**Description**

Field

The present invention relates to a pulse application method and a pulse application device. Background
For example, coagulation treatment in which collagen fibers are heated to coagulate the collagen fibers by application of laser light to skin has been performed as skin treatment.

Citation List

Patent Literature

Non Patent Literature 1: Current Laser Resurfacing Technologies: A Review that Delves Beneath the Surface, Jason Preissig, Kristy Hamilton, Ramsey Markus, Seminars in Plastic Surgery, Vol. 26, No.3, 2012, PP. 109-116

Summary

Technical Problem

When living tissue, particularly, skin is heated by laser light application, however, similar heating occurs not only in collagen fibers but also in cells in the living tissue and therefore there is a risk that it is hard to inhibit side effects.
Thus, an object of the present invention is to obtain a pulse application method and a pulse application device that make it possible to selectively heat collagen fibers in living tissue while inhibiting heating of cells that are present in the living tissue.

Solution to Problem

A pulse application method according to the present invention is a pulse application method of applying a pulse of light to living tissue to heat the living tissue, wherein a wavelength of the light is set within a range in which a temperature rise width of collagen fibers in the living tissue when the light is applied to the living tissue is larger than a temperature rise width of water containing cells that are contained in the living tissue and that are present around the collagen fibers.
In the pulse application method, for example, the cells are fibroblast.
In the pulse application method, for example, an application time period in which a pulse of light is applied to the living tissue and a non-application time period in which no pulse of light is applied to the living tissue are repeated alternately.
In the pulse application method, for example, the non-application time period is set such that a temperature of the water at an end of the non-application time period is equal to or lower than a first temperature.
In the pulse application method, for example, the non-application time period is set such that the temperature of the water at the end of the non-application time period is approximately equal to the temperature of the water at a start of the application time period immediately before the non-application time period.
In the pulse application method, for example, the non-application time period is set such that a temperature of the collagen fibers at an end of the non-application time period is higher than the temperature of the collagen fibers at a start of the application time period immediately before the non-application time period.
In the pulse application method, for example, the non-application time period is set based on a thermal relaxation time period from an end of the application time period immediately before the non-application time period until a time when a temperature of the collagen fibers decreases to a temperature obtained by adding a temperature width obtained by dividing the temperature rise width of the collagen fibers in the application time period by a bottom of a natural logarithm to a temperature at a start of the application time period.
In the pulse application method, for example, the non-application time period is set equal to or more than the thermal relaxation time period.
In the pulse application method, for example, the non-application time period is between 80 [ms] and 210 [ms] inclusive.
In the pulse application method, for example, the wavelength and an application time period of the pulse are set such that the temperature of the water at an end of the application time period is equal to or lower than a second temperature.
In the pulse application method, for example, the second temperature is a thermal denaturation threshold temperature at which collagen molecules in collagen fibers thermally denature.
In the pulse application method, for example, the second temperature is a reversible thermal denaturation threshold temperature at which collagen molecules in collagen fibers reversibly thermally denature.

In the pulse application method, for example, the wavelength and an application time period of the pulse are set such that collagen molecules in the collagen fibers thermally denature and the cells are not thermally damaged.

In the pulse application method, for example, the wavelength and an application time period of the pulse are set such that collagen molecules in the collagen fibers thermally denature and the cells are reversibly thermally damaged.

In the pulse application method, for example, the wavelength and an application time period of the pulse are set such that collagen molecules in the collagen fibers irreversibly thermally denature and the cells are not thermally damaged.

In the pulse application method, for example, the wavelength and an application time period of the pulse are set such that collagen molecules in the collagen fibers irreversibly thermally denature and the cells are reversibly thermally damaged.

In the pulse application method, for example, the wavelength and an application time period of the pulse are set such that thermal transpiration occurs on a surface of the living tissue.

In the pulse application method , for example, the application time period in which the pulse of light is applied to the living tissue and the non-application time period in which no pulse of light is applied to the living tissue are repeated alternately, and the wavelength, the application time period, the non-application time period, and the number of times the application time period is repeated are set such that collagen molecules in the collagen fibers thermally denature and the cells are not thermally damaged.

In the pulse application method, for example, the application time period in which the pulse of light is applied to the living tissue and the non-application time period in which no pulse of light is applied to the living tissue are repeated alternately, and the wavelength, the application time period, the non-application time period, and the number of times the application time period is repeated are set such that collagen molecules in the collagen fibers thermally denature and the cells are reversibly thermally damaged.

In the pulse application method, for example, the application time period in which the pulse of light is applied to the living tissue and the non-application time period in which no pulse of light is applied to the living tissue are repeated alternately, and the wavelength, the application time period, the non-application time period, and the number of times the application time period is repeated are set such that collagen molecules in the collagen fibers irreversibly thermally denature and the cells are not thermally damaged.

In the pulse application method, for example, the application time period in which the pulse of light is applied to the living tissue and the non-application time period in which no pulse of light is applied to the living tissue are repeated alternately, and the wavelength, the application time period, the non-application time period, and the number of times the application time period is repeated are set such that collagen molecules in the collagen fibers irreversibly thermally denature and the cells are reversibly thermally damaged.

In the pulse application method, for example, the application time period in which the pulse of light is applied to the living tissue and the non-application time period in which no pulse of light is applied to the living tissue are repeated alternately, and the wavelength, the application time period, the non-application time period, and the number of times the application time period is repeated are set such that thermal transpiration occurs on a surface of the living tissue.

In the pulse application method, for example, a ratio of the temperature rise width of the collagen fibers to the temperature rise width of the water when the light is applied to the living tissue is equal to or larger than 1.1.

In the pulse application method, for example, a denaturation state of the collagen fibers is detected, and at a time when a given denaturation state of the collagen fibers is detected, application of the pulse ends.

In the pulse application method, for example, in an application time period of the pulse, a plurality of pulses of light are applied intermittently.

In the pulse application method, for example, the wavelength of the light is set at a wavelength at which an absorption coefficient of the collagen fibers is larger than an absorption coefficient of the water.

The pulse application method, for example, the wavelength of the light is set at a value longer than 1480 [nm].

In the pulse application method, for example, the wavelength of the light is set at a value equal to or less than 1600 [nm].

A pulse application device according to the present invention is a pulse application device configured to apply a pulse of light to living tissue to heat the living tissue, wherein a wavelength of the light is set within a range in which a temperature rise width of collagen fibers in the living tissue when the light is applied to the living tissue is larger than a temperature rise width of water containing fibroblast that are contained in the living tissue and that are present around the collagen fibers.

The pulse application device, for example, includes a detector configured to detect a denaturation state of the collagen fibers, wherein at a time when a given denaturation state of the collagen fibers is detected by the detector, application of the pulse ends.

In the pulse application device, for example, the wavelength of the light is set at a value longer than 1480 [nm].

In the pulse application device, for example, the wavelength of the light is set at a value equal to or less than 1600 [nm].

Advantageous Effects of Invention

According to the pulse application method and the pulse application device, it is possible to selectively heat collagen fibers in living tissue while inhibiting heating of cells that are present in the living tissue.

Brief Description of Drawings

FIG. 1 is an exemplary and schematic configuration diagram of a pulse application device according to an embodiment.
FIG. 2 is a schematic diagram illustrating an internal structure of living tissue.
FIG. 3 is an exemplary and schematic graph illustrating wavelength spectra of absorption coefficients of water and collagen molecules and a ratio of the absorption coefficient of collagen molecules to that of water.
FIG. 4 is an exemplary and schematic timing chart illustrating changes of water and collagen fibers in temperature over time caused by a pulse application method according to the embodiment.
FIG. 5 is an exemplary graph illustrating a correlation between a pulse application time period and a ratio of the rise of the temperature of collagen fibers to that of water in application of one pulse in the pulse application time period in the pulse application method according to the embodiment.
FIG. 6 is an exemplary graph illustrating pulse non-application time periods with respect to respective pulse application time periods in the pulse application method according to the embodiment, which are pulse non-application time periods enabling the temperature of water at the end of the pulse non-application time period to be equal to the temperature at the start of the pulse application time period.

Description of Embodiments

An exemplary embodiment of the present invention and modifications thereof will be disclosed below. Configurations of the embodiment and the modification presented below and the functions and results (effects) brought by the configurations are an example. The embodiment can be realized by a configuration other than the configurations disclosed by the embodiment and the modifications below. According to the present invention, it is possible to obtain at least one of various effects (including derivative effects) obtained by the configurations described below.

Embodiment

Configuration of Pulse Application Device

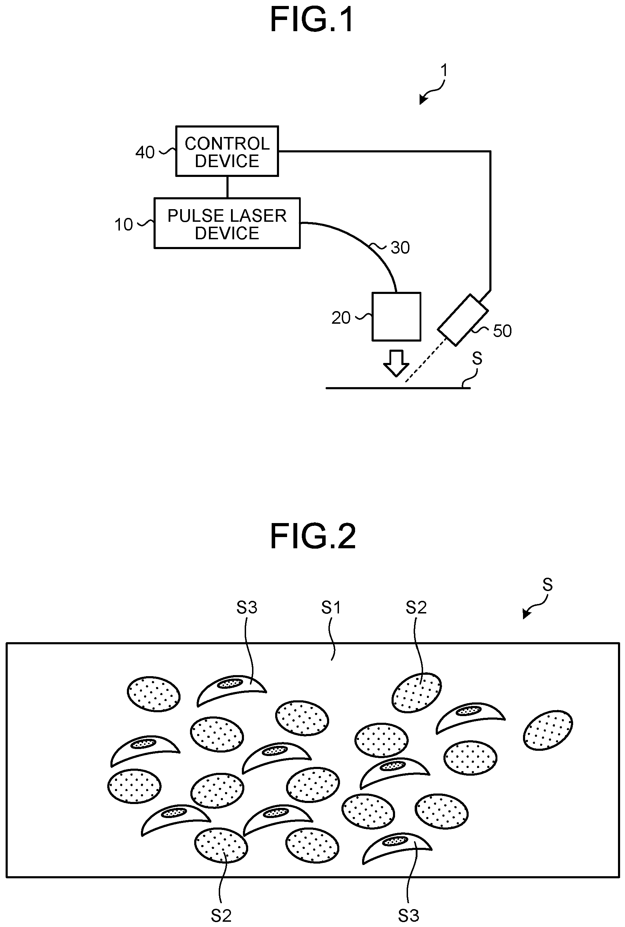

FIG. 1 is a configuration diagram of a pulse application device 1. As illustrated in FIG. 1, the pulse application device 1 includes a pulse laser device 10, an optical head 20, an optical fiber 30, a control device 40, and a sensor 50.
The pulse laser device 10 is capable of emitting a pulse of infrared laser light. The pulse of laser light that is emitted by the pulse laser device 10 is transmitted to the optical head 20 via the optical fiber 30 and is applied from the optical head 20 to skin S. The optical head 20 disperses and collimates or focuses the pulse of laser light and applies the pulse of laser light to the skin S. The skin S is heated and/or stimulated by the applied pulse of laser light. The optical head 20 is moved manually or a robot arm, or the like, making it possible to apply the pulse of laser light to a desired spot on the skin S. The control device 40 is able to set or change a specification, such as an intensity of a pulse of laser light that is emitted from the pulse laser device 10, a pulse application time period (a pulse ON period or a pulse width) of the laser light and a pulse non-application time period (pulse OFF period) of the laser light. The pulse laser device 10 may include a fiber laser device or a semiconductor laser device. The skin S is an example of living tissue.
The sensor 50 detects a thermal denaturation state of collagen fibers that are contained in the skin S. The thermal denaturation state of collagen fibers will be described, exemplifying a cornea. A cornea is mainly composed of collagen and, because collagen fibers are aligned in the cornea, the cornea has high transparency in the state of not thermally denaturing. When a structure change occurs in collagen in the cornea due to thermal denaturation, the structurally changed part becomes the center of scattering and the cornea becomes cloudy in a visible light region. Based on the same principle, it is possible to detect a thermal denaturation state of the skin S by measuring a scattering state by the optical sensor 50, for example, measuring back scattering attenuation of irradiation light. In this case, the sensor 50 is a noncontact sensor. When the skin S thermally denatures, the hardness of the skin S increases. Thus, measurement of hardness by the mechanical sensor 50, such as a hardness sensor, enables detection of a thermal denaturation state of the skin. In this case, the sensor 50 is a contact sensor. It is possible to detect a thermal denaturation state of collagen molecules not only in a cornea but also in other tissue. The control device 40 is able to end application of pulses at a time when the sensor 50 detects a given thermal denaturation state of collagen fibers. The sensor 50 is an example a detector.

Tissue of Skin (Living Body)

FIG. 2 is a schematic diagram illustrating an internal structure of living tissue, such as the skin S. As illustrated in FIG. 2, the skin S contains living organisms water S1, collagen fiber bundles S2 that are bundles of collagen fibers, and fibroblast S3. The fibroblast S3 generate collagen fibers. Light absorption properties and heat conduction properties of the fibroblast S3 are approximately the same as those of water. Thus, in analysis of changes in temperature, the fibroblast S3 can be regarded as water. The fibroblast S3 are an example of cells.

Setting of Wavelength of Laser Light

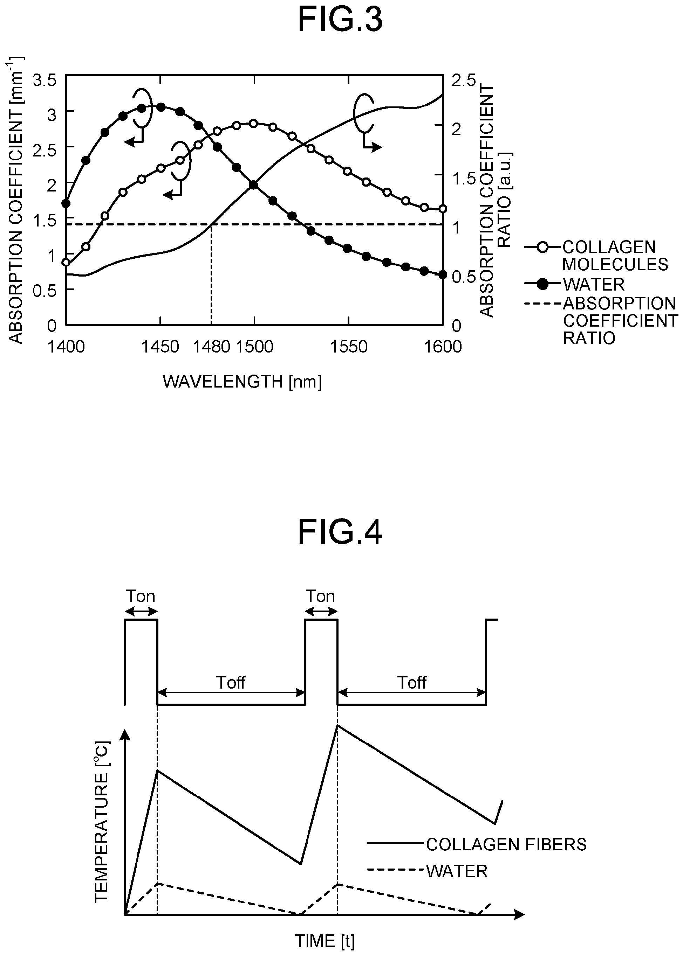

FIG. 3 is a graph illustrating wavelength spectra of absorption coefficients of water and collagen molecules and a ratio of the absorption coefficient of collagen molecules to that of water. It has been proved that, as illustrated in FIG. 3, in the range of near-infrared light, an absorption coefficient of water and an absorption coefficient of collagen molecules vary depending on the wavelength (Banri Ono et. al, The Journal of Japan Society for Laser Surgery and Medicine, 36, 324, 2015 and Kou, et al, Appl Opt, 32, 3521-3540, 1993). The absorption coefficient of water is, in a range from the wavelength of irradiation light of 1400 [nm] to 1600 [nm], at peak when the wavelength is approximately 1450 [nm], decreases as the wavelength becomes shorter than approximately 1450 [nm] and decreases as the wavelength becomes longer than approximately 1450 [nm]. On the other hand, the absorption coefficient of collagen molecules is, in a range of the wavelength of irradiation light from 1400 [nm] to 1600 [nm], at peak when the wavelength is approximately 1500 [nm], decreases as the wavelength becomes shorter than approximately 1500 [nm], and decreases as the wavelength becomes longer than approximately 1500 [nm]. Accordingly, as illustrated in FIG. 3, in the range of the wavelength of irradiation light from 1400 [nm] to 1600 [nm], the ratio of the absorption coefficient of collagen molecules to the absorption coefficient of water (referred to as an absorption coefficient ratio below) increases as the wavelength becomes longer. Furthermore, it is estimated that the absorption coefficient of collagen molecules further decreases when the wavelength becomes longer than 1600 [nm].

Based on such nature, in the present embodiment, the wavelength of laser light to be applied to the skin S, that is, the wavelength of laser light that the pulse laser device 10 emits is set within a range in which the absorption coefficient of collagen molecules is larger than the absorption coefficient of water, that is, is set longer than 1480 [nm]. It can be argued that the range in which the wavelength is longer than 1480 [nm] is a range in which collagen fibers (collagen fiber bundles) are heated more easily than water.

As described above, in the range in which the wavelength is longer than 1600 [nm], the absorption coefficient of collagen molecules decreases as the wavelength becomes longer and this means that an optical energy absorption efficiency of collagen molecules decreases depending on application of laser light whose wavelength is longer than 1600 [nm]. Thus, in the present embodiment, the wavelength of laser light that is applied to the skin S, that is, the wavelength of laser light that the pulse laser device 10 emits is set equal to or under 1600 [nm].

From such consideration, in the present embodiment, the wavelength of laser light that is applied to the skin S, that is, the wavelength of laser light that the pulse laser device 10 emits is set longer than 1480 [nm] and equal to or under 1600 [nm].

Setting of Pulse Application time period and Pulse Non-Application time period

FIG. 4 is a schematic timing chart illustrating changes of water and collagen fibers in temperature over time associated with application of pulses of laser light.

As illustrated in FIG. 4, in a pulse application time period Ton, the temperatures of water and collagen fibers increase because of absorption of energy of laser light. As described above, in the pulse application time period Ton, because the wavelength of laser light is set at a wavelength at which the absorption coefficient of collagen fibers is larger than the absorption coefficient of water, a rise of the temperature of collagen fibers (a temperature rise width) is larger than a rise of the temperature of water (a temperature rise width). In other words, the wavelength of laser light is set such that the rise of the temperature of collagen fibers during the pulse application time period Ton is larger than the rise of the temperature of water during the pulse application time period Ton.

The inventors focused on the aspect that the temperature of collagen fibers rises during the pulse application time period Ton and calculated a pulse application time period Ton makes it possible to further increase the absorption coefficient ratio.

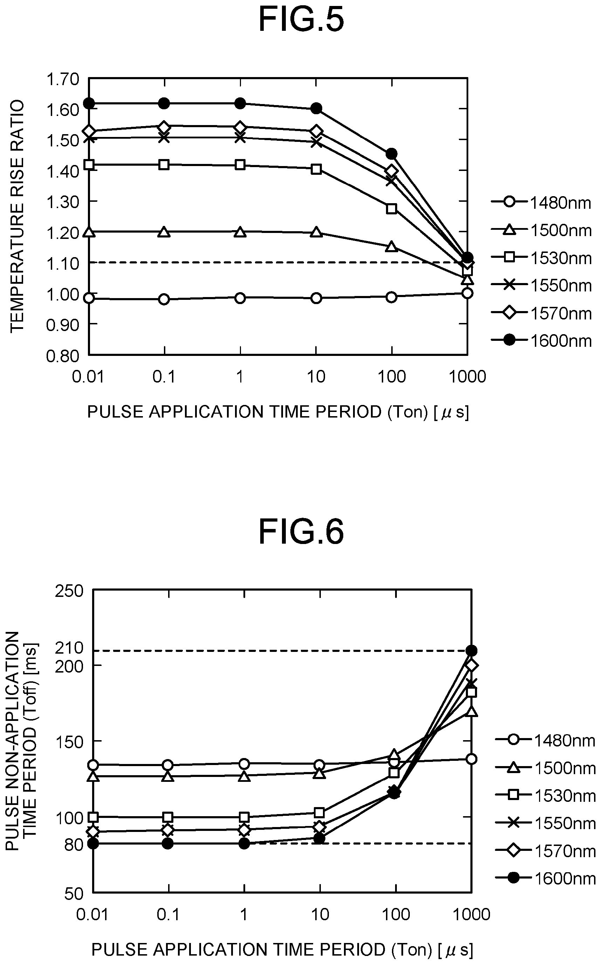

FIG. 5 is a graph illustrating a correlation between the pulse application time period Ton and the ratio of the rise of the temperature of collagen fibers to that of water (simply referred to as the temperature rise ratio below) in application of a single pulse during the pulse application time period Ton. FIG. 5 is of calculation values by numerical simulation with respect to a calculation model obtained by modeling subcutaneous tissue. In the numerical simulation, parameters, such as a border condition and a coefficient, are adjusted such that the calculated value of thermal denaturation depth obtained by application of laser light is approximate to an experimental value of thermal denaturation depth obtained by application of laser light to a sample of subcutaneous tissue. In this numerical simulation, irradiation energy per pulse does not depend on the pulse application time period Ton and is constant. In other words, the smaller the pulse application time period Ton is, the larger the applied energy per unit time is and, the longer the pulse application time period Ton is, the smaller the irradiation energy per unit time is. This simulation makes it possible to estimate a temperature of each part at the time of application of laser light and changes in temperature over time with respect to various sites of irradiation, application environments, and application conditions. The point in which the temperatures of water and fibroblast are measured is set in a position between collagen fibers that are adjacent to each other with an interval (intermediate position), which is a position less susceptible to thermal absorption by collagen fibers.

As illustrated in FIG. 5, it was proved that, as for the range of wavelength of laser light where the absorption coefficient ratio is larger than 1, specifically, in the range of 1480 [nm] or longer, the temperature rise ratio can be kept larger than 1 within the range in which the pulse application time period Ton is between 0.01 [μs] and 1000 [μs] inclusive.

On the other hand, as illustrated in FIG. 4, in the pulse non-application time period Toff, as illustrated in FIG. 4, because of transmission of heat from collagen fibers and water whose temperatures become higher than the surroundings (the air) to the surroundings, the temperatures of water and collagen fibers decrease. The decrease of the temperature of water and the decrease of the temperature of collagen fibers depend on the specific heat of water and collagen fibers.

The inventors focused on the aspect that the temperature of water decreases in the pulse non-application time period Toff and calculates the pulse non-application time period Toff that makes it possible to selectively heat collagen fibers while inhibiting the temperature of water from rising.

FIG. 6 is a graph illustrating pulse non-application time periods Toff with respect to respective pulse application time periods Ton in the same numerical simulation as that in which the result in FIG. 5 is obtained, which are pulse non-application time periods enabling the temperature of water at the end of the pulse non-application time period Toff to be equal (to decrease) to the temperature at the start of the pulse application time period Ton immediately before the pulse non-application time period Toff. In the example in FIG. 6, the temperature of water at the start of the pulse application time period Ton is an example of a first temperature.

It was proved that, as illustrated in FIG. 6, setting the pulse-non-application time period Toff according to the wavelength of laser light and the pulse application time period Ton enables the temperature of water at the end of the pulse non-application time period Toff to decrease to a temperature (first temperature) approximately equal to the temperature that at the start of the pulse application time period Ton. As understood from FIG. 6, it is preferable that the pulse non-application time period Toff be between 80 [ms] and 210 [ms] inclusive.

It can be understood that, as illustrated in FIG. 4, in the case where the temperature of collagen fibers at the end of the pulse non-application time period Toff is higher than the temperature of water, repeating application of a pulse, in other words, repeating the pulse-application time period Ton and the pulse non-application time period Toff gradually increases the difference in temperature between water and collagen fibers at the end of the pulse non-application time period Toff and eventually it is possible to more selectively heat collagen fibers.

It is preferable that the pulse non-application time period Toff be set according to a thermal relaxation time period of collagen fibers. The thermal relaxation time period of collagen fibers may be defined as a time period from the end of the pulse application time period Ton immediately before the pulse non-application time period Toff until a time when the temperature of collagen fibers decreases to a temperature obtained by adding a temperature width ($\Delta$tc/e) obtained by dividing a temperature rise width of collagen fibers in the pulse application time period Ton by a bottom of a natural logarithm to the temperature at the start of the pulse application time period Ton. Here, $\Delta$tc is the temperature rise width of collagen fibers.

The pulse non-application time period Toff can be set longer than the thermal relaxation time period of collagen fibers. In an example, the pulse non-application time period Toff may be set at a value obtained by multiplying the thermal relaxation time period by a coefficient larger than 1 (for example, 1.1, or the like). In this case, the temperature of water at the end of the pulse non-application time period Toff tends to be equal to or lower than the temperature of water at the start of the pulse application time period Ton immediately before the pulse non-application time period Toff. This makes it possible to assuredly inhibit the temperature of water from rising.

The pulse non-application time period Toff can be set equal to the thermal relaxation time period of collagen fibers. In this case, the temperature of water at the end of the pulse non-application time period Toff is equal the temperature of water at the start of the pulse application time period Ton immediately previous to the pulse non-application time period Toff. It is possible also in this case to inhibit the temperature of water from rising.

The pulse non-application time period Toff can be set shorter than the thermal relaxation time period of collagen fibers. In an example, the pulse non-application time period Toff may be set at a value obtained by multiplying the thermal relaxation time period by a coefficient (for example, 0.9, or the like) larger than 0 and smaller than 1. In this case, the temperature of water at the end of the pulse non-application time period Toff is higher than the temperature of water at the start of the pulse application time period Ton immediately before the pulse non-application time period Toff. In other words, it is higher than the temperature of water at the end of the pulse non-application time period Toff presented in FIG. 4. This enables the temperature of collagen fibers at the end of the pulse non-application time period Toff to be higher and therefore enables the temperature of collagen fibers more speedily. Note that, in this case, because the temperature of water rises gradually according to application of pulse, the temperature of water at the end of the pulse non-application time period Toff is set such that, for example, the temperature rise with respect to the temperature of water at the start of the initial pulse application time period Ton is equal to or smaller than a given value or may be set equal to or smaller than a temperature that does not damage the skin S. A temperature obtained by adding a given value to the temperature of water at the start of the initial pulse application time period Ton or the temperature that does not damage the skin S is an example of the first temperature. The first temperature is a threshold temperature that is set according to the temperature of water at the end of the pulse non-application time period Toff.

Furthermore, regardless of the length of the pulse non-application time period Toff, the highest temperature of water in the pulse application time period Ton is set, for example, equal to or smaller than a temperature that does not damage the skin S. The temperature that does not damage the skin S in this case is an example of a second temperature. The second temperature is a threshold temperature that is set according to the temperature of water at the end of the pulse application time period Ton.

Normally, the higher the temperature is, the shorter the time in which protein thermally denatures is and, the lower the temperature is, the longer the time in which protein thermally denatures is. The protein of which a living organism consists causes an irreversible thermal denaturation at approximately 60 °C. In some cases, although it varies depending on the site or internal organ and varies depending on the heating time, thermal damage may occur in living tissue even at approximately 45 °C when the heating time is relatively long. It is possible to acquire a threshold temperature corresponding to a site of irradiation, an irradiation environment, an irradiation condition, etc., experimentally in advance (refer to Literature 2: Minoru Obara, Tsunenori Arai, Katsumi Midorikawa. Applied Laser Engineering. Corona Publishing Co., Ltd., 1998.)

The thermal relaxation time period varies depending on the water content of the skin S, arrangement of collagen fibers (fiber bundle of collagen fibers) in the skin S, the intensity of laser light, the pulse application time period Ton, etc. Thus, the thermal relaxation time period and the pulse non-application time period Toff may be set variably according to the water content of the skin S, arrangement of collagen fibers in the skin S, the intensity of laser light, the pulse application time period Ton, etc.

The pulse application time period Ton, the pulse non-application time period Toff and the energy (intensity) applied in the pulse application time period Ton may be the same among multiple pulses or may differ per pulse. For example, the control device 40 may change at least one of the pulse application time period Ton, the pulse non-application time period Toff, and the energy according to the denaturation caused by heating of collagen fibers.

During the pulse application time period Ton, the pulse of laser light may be applied intermittently like a burst waveform. The time of intermittence in this case is, for example, sufficiently shorter than the thermal relaxation time period.

In order to obtain the effect of a single pulse, the wavelength of laser light and the pulse application time period Ton are set such that the collagen molecules in collagen fibers thermally denature. In order to obtain the effect of multiple pulses, the wavelength of laser light, the pulse application time period Ton, the pulse non-application time period Toff, and the number of times the pulse application time period Ton is repeated are set such that collagen molecules in collagen fibers thermally denature.

Locally and selectively heating living tissue by repetitive application of pulses of laser is a method of, when the absorption coefficient of tissue to be heated selectively is larger than the absorption coefficient of surrounding tissue, selectively heating tissue with a large absorption coefficient by adjusting the pulse width (the pulse application time period Ton) and the pulse interval (the pulse non-application time period Toff). This method is referred to as selective photothermolysis (refer to Literature 3: Selective photothermolysis: precise microsurgery by selective absorption of pulsed radiation, RR Anderson, JA Parrish, Science, Vol. 220, Issue 4596, PP. 524-527, 1983).

Specifically, adjustments of the pulse width and the pulse interval are made in consideration of heat conduction to the surroundings in the pulse interval. The state of heat conduction to the surroundings is determined by the heat conductivity and the shape of a heat generating part. In order to simply argue the issue of unsteady heat conduction, a feature heat conduction (thermal relaxation constant) is defined and used. For selective photothermolysis, the pulse width is set sufficiently shorter than the feature heat conduction time and the pulse interval is set sufficiently longer than the feature heat conduction time (Literature 2).

Selective photothermolysis does not give a heat generation contrast equal to or more than the absorption ratio between a living tissue part whose light absorption is large and the surrounding living tissue whose light absorption is small (in other words, the heat generation ratio depending on laser application) and the absorption ratio gives an upper limit of the heat generation ratio. On the contrary, when a sequential laser is applied for a long time, because the heat conduction leads to uniform heating, local and selective heating cannot be realized.

Thermal denaturation of collagen molecules includes irreversible thermal denaturation and reversible thermal denaturation. Irreversible thermal denaturation causes coagulation of collagen molecules and is used for treatment of cancers, coagulation of retina, hemostasis, etc. Reversible thermal denaturation of collagen molecules is used to weld a blood vessel, the small intestine, skin, etc.

Collagen molecules form three-dimensional spiral structure chains; however, the hydrogen bonds that bind the spiral structure chains are disconnected with an increase in temperature and, when overheated, the collage molecules are dispersed and shrink. This state is irreversible thermal denaturation. In the case of denaturation with a small ratio of disconnection of bonds, a return to the original structure is made in some cases and this transient thermal denaturation is referred to as reversible thermal denaturation. Collagen molecules are classified into a few tens of types according to the types and shapes of amino acid contained and the site of presence in living tissue differs. In the present embodiment, I or III collagen serving as main components of skin, skin loss repair, bone, etc., is main referred to. The physical properties of collagen molecules differ also depending on animal species and thermal properties differ depending on the environment in which the living organism lives (refer to Literature 4: On a Relationship Between the Arrhenius Parameters from Thermal Damage Studies, Neil T. Wright, Journal of Biomechanical Engineering, Vol. 125, No. 2, PP. 300-304, 2003). In the present embodiment, thermal properties of collagen molecules of mammals including human beings living on land are mainly referred to.

Thermal denaturation of collagen molecules is one type a chemical reaction process and is described according to Arrhenius theory (Equation (1), Arrhenius equation) describing a chemical reaction (refer to Literature 5: Finite element analysis of temperature controlled coagulation in laser irradiated tissue, T. N. Glenn, S. Rastegar, S. L. Jacques, IEEE Transactions on Biomedical Engineering, Vol. 43, No. 1, PP 79-87, Jan. 1996, Eq(3)).

$$\Omega = A\int_0^{\Delta t} \exp\left(-\frac{E_a}{RT}\right)dt \qquad\qquad (1)$$

where $\Omega$: stored heat denaturation amount, A: frequency factor [1/s], $\Delta t$: heating time [s], $E_a$: activation energy [J/mol], R: gas constant [J/mol·K]($\approx$8.314), and T: temperature [K]. The stored heat denaturation amount is a guide of denaturation and, in general, at approximately 1, it is determined that it is thermal denaturation.

From Equation (1), when the heating time is constant, the stored heat denaturation amount is proportional to the time. The power of an index function is a negative number and the frequency factor and the stored heat denaturation amount are constants that are determined by the substance and thus, the higher the temperature is, the larger the value of the exponential function is. Accordingly, the thermal denaturation depends on the heating time and the holding time.

The fact that the stored heat denaturation amount is a guide of a denaturation amount and does not provide a precise threshold is taken as the reason why the stored heat denaturation amount based on Arrhenius theory is not used in general. Furthermore, the frequency factor and the activation energy vary depending on the substance (protein). As described above, while Arrhenius theory can give a theoretical support on living organism protein coagulation, standing on precise Arrhenius theory has less meaning in a practical broad argument. Thus, in general, a determination is made based on a thermal denaturation temperature serving as a guide (refer to Literature 6: Photophysical processes in recent medical laser developments: A review, Jean-Luc Boulnois, Lasers in Medical Science, January 1986, Volume 1, Issue 1, PP. 47-66) .

A practical heat application time in a spot in living organism treatment is short because it is a local action particularly in an operation treatment device and does not exceed, for example, one second. In a burn that is a typical living organism thermal damage, heating does not continue because of a living biology reaction response and, typically, it is approximately 50 ms. As described above, as a result of limitation of on the heating time in treatment, a temperature that gives a sufficient accumulated thermal denaturation amount is determined roughly.

As described above, it is difficult to precisely obtain a temperature and a heating time that are necessary to obtain irreversible thermal denaturation of living tissue and a guide value of a temperature enabling irreversible thermal denaturation is used practically (refer to Literature 6). In the present embodiment, a generally-used guide value of a temperature enabling irreversible thermal denaturation is used as a threshold temperature. When a thermal denaturation threshold temperature is exceeded, protein thermally denatures and, when a reversible thermal denaturation threshold temperature is exceeded, protein reversibly thermally denatures.

Fibroblast are cells that generate collagen molecules and thus it is preferable that the temperature of fibroblast, that is, the temperature of water be a temperature at which fibroblast are not damaged, that is, a temperature causing no thermal denaturation. As for fibroblast, it has been proved that activities are activated by heating fibroblast to an extent where the fibroblast are not damaged to cause the fibroblast to reversibly thermally denature.

When protein in fibroblast cause irreversible thermal denaturation, vital activity stops and the cells necrose (thermal damage or irreversible thermal damage). On the other hand, when reversible thermal denaturation of protein or significantly little and local irreversible denaturation of protein occurs, in some cases, homeostasis causes restoration outcome (activation) of the fibroblast (reversible damage). Fibroblast may go into necrosis because cytoplasm goes out of the cells via pores that are opened due to thermal damage of the surrounding phospholipid bilayer and the surrounding body fluid flows into the cells via the cell membrane and causes the cells to swell and the cells rupture. In thermal coagulation treatment, formation of pores in the cell membrane and thermal denaturation of protein are caused and thresholds thereof remain a subject of dispute. Thermal denaturation of protein tends to be used for general modeling.

Selective heating can be executed under a condition that causes thermal transpiration on the surface of living tissue. Thermal transpiration of living tissue occurs at the moment when the temperature of moisture in living tissue reaches 100 °C locally and boils. When water in the liquid phase turns into steam, the volume expands approximately thousandfold, the cell membrane is torn and the tissue disappears. In order to cause this to occur successively, a condition that sequentially supplies latent heat that is lost due to boiling can be set (refer to Literature 2).

In order to obtain an effect of a single pulse, the wavelength of laser light and the pulse application time period Ton are set such that fibroblast are not thermally damaged or are reversibly thermally damaged. In order to obtain the effect of multiple pulses, the wavelength of laser light, the pulse application time period Ton, the pulse non-application time period Toff, and the number of times the pulse application time period Ton is repeated are set such that fibroblast are not thermally damaged or are reversibly thermally damaged.

In the present embodiment, the longer the pulse application time period Ton is, the higher the temperatures of collagen fibers and water (fibroblast) increase and, the longer the pulse non-application time period Toff is, the lower the temperatures of collagen fibers and water (fibroblast) are. The difference of the temperature of collagen fibers after the end of the pulse non-application time period Toff from the temperature of collagen fibers at the start of the pulse application time period Ton and the difference of the temperature of water (fibroblast) at the end of the pulse non-application time period Toff from the temperature of water (fibroblast) at the start of the pulse application time period Ton is adjustable by the wavelength, the pulse application time period Ton and the pulse non-application time period Toff. The number of times the pulse application time period Ton is repeated (also simply referred to as a repetition number-of-times below) can be set based on the defenses in temperature thereof. For example, in the situation where each of the temperatures increases in each set of the pulse application time period Ton and the pulse non-application time period Toff, the larger the difference in temperature is, the smaller the repetition number-of-times until the time when each of the temperatures reaches a given temperature is and, the smaller the difference in temperature is, the larger the repetition number-of-times until the time when each of the temperatures reaches the given temperature is. The wavelength of laser light, the pulse application time period Ton, the pulse non-application time period Toff, and the repetition number-of-times can be set in consideration of an environmental condition, such as the ambient temperature or the body temperature. Note that the lengths of the pulse application time period Ton and the pulse non-application time period Toff need not be set the same (at constant values) with respect to a plurality of sets of pulse application and the lengths may be switched when it comes close to a given temperature or may be changed per pulse.

In the pulse application device 1 illustrated in FIG. 1, the optical fiber 30 and the optical head 20 may be replaced with a space optical system. In this case, the space optical system can include a lens, a mirror, etc. The space optical system may include a Galvano scanner. Laser light can be applied by the Galvano scanner to a desired spot on the skin S. Furthermore, a space optical system including a Galvano scanner may be arranged on a light output side of the optical head 20 of the pulse application device 1.

For example, when high speed scanning of laser light is made with the Galvano scanner in the space optical system, laser light is applied to a certain point on the skin S for a period corresponding to a scanning speed. In this case, even when the laser light that is emitted by the pulse laser device 10 is a continuous wave, it is possible to apply the laser light to the skin S in a manner of pulses. For example, the trajectory of laser light by scanning may be in a shape of a ring. Accordingly, laser light is applied in a manner of pulses repeatedly to sequential points on the skin S and, even when laser light is a continuous wave, it is possible to control heat input to the skin S over time.

A control mechanism for controlling a beam profile of laser light may be further added to the pulse application device 1. As such a control mechanism, a mechanism that includes a special optical fiber and that generates a beam that is a combination of a beam of a single-peak-type profile and a beam of a ring-type profile is known. As another control mechanism, a mechanism including a spatial light modulator (SLM) is known. The SLM is, for example, a space phase modulation device that consists of pixels of a phase modulation device that are a plurality of micro-optical operation devices that are arrayed one-dimensionally or two-dimensionally and that controls the beam profile of input laser light by electrically controlling the phase of each of the pixels.

Furthermore, as another control mechanism, a mechanisms including a DOE (diffractive optical element) is known. The DOE is a diffractive optical element configured by integrating a plurality of diffraction gratings with different periods. The DOE is capable of forming a beam shape by deflecting input laser light to a direction of an effect of each diffraction grating or superimposing the laser light. For example, the DOE is capable of forming the input single-peak-type beam into a line beam or a ring beam.

The pulse application device 1 includes the single pulse laser device 10; however, the pulse application device 1 may include a plurality of the pulse laser devices 10. When a plurality of the pulse laser devices 10 are included, the wavelengths of laser light to be emitted may be all the same or may differ at least partly.

The pulse application device 1 may include a reference laser device that emits laser light of a visible light area in addition to the pulse laser device 10 that emits near-infrared laser light. The reference laser device may be configured to emit reference laser light (continuous wave or pulse) in the visible light region to apply the laser light to the same position as the position on the skin S in which the laser light from the pulse laser device 10 is applied. The reference laser light can be checked visually, which make it easy to check the position on the skin S in which the laser light from the pulse laser device 10 is applied.

The sensor 50 may be an infrared thermometer, a thermography, a color sensor, an image sensor, an acoustic sensor, a power meter, or the like. The pulse application device 1 may include a sensor system including the sensor 50 and a measurement device, such as a spectrum analyzer. For example, the control device 40 may be configured to be capable of executing a computer program enabling arithmetic processing using a trained model that is generated by machine learning, or the like, previously. In this case, the control device 40 may perform arithmetic processing on data that is acquired by the sensor 50 using the trained model and determine a thermal denaturation state of collagen fibers contained in the skin S.

As described above, in the present embodiment, the wavelength of laser light (light) is set within a range in which the temperature rise width of collagen fibers in the skin S when the laser light is applied to the skin S (living tissue) is larger than the temperature rise width of water containing fibroblast (cells) that are contained in the skin and that are present around the collagen fibers.

According to the present embodiment, in the pulse application time period Ton, a rise of the temperature of collagen fibers is set larger than a rise of the temperature of water, which makes it possible to heat collagen fibers more efficiently. Thus, according to such a method, for example, it is possible to obtain more efficient treatment effects than by coagulation of collagen fibers.

In the present embodiment, for example, the cells are fibroblast.

The method of the present embodiment is effective in the case where the cells are fibroblast.

In the present embodiment, for example, the pulse application time period Ton in which a pulse of light is applied to the skin S and the pulse non-application time period Toff in which no pulse of light is applied to the skin S are repeated alternately.

Accordingly, in the pulse non-application time period Toff, it is possible to inhibit the temperature of fibroblast contained in water from rising and eventually inhibit fibroblast from being damaged.

In the present embodiment, for example, the pulse non-application time period Toff (non-application time period) is set such that the temperature of water at the end of the pulse non-application time period Toff is equal to or lower than the first temperature (given temperature).

Accordingly, it is possible more assuredly inhibit a temperature rise of fibroblast and eventually a damage of the fibroblast.

In the pulse application method of the present embodiment, for example, the pulse non-application time period Toff is set such that the temperature of water at the end of the pulse non-application time period Toff is approximately equal to the temperature of water at the start of the pulse application time period Ton.

Accordingly, it is possible to more assuredly inhibit a temperature rise of fibroblast and eventually a damage of fibroblast.

In the present embodiment, the pulse non-application time period Toff is set such that the temperature of collagen fibers at the end of the pulse non-application time period Toff is higher than the temperature of collagen fibers at the start of the pulse application time period Ton immediately before the pulse non-application time period Toff.

Accordingly, it is possible to speedily increase the temperature of collagen fibers by repeating application of a pulse.

In the present embodiment, the pulse non-application time period Toff is set based on the thermal relaxation time period from the end of the pulse application time period Ton immediately before the pulse non-application time period Toff until a time when the temperature of collagen fibers decreases to a temperature obtained by adding the temperature rise width obtained by adding the temperature width obtained by dividing the temperature rise of collagen fibers in the pulse application time period Ton by the bottom of a natural logarithm to the temperature at the start of the pulse application time period Ton.

Accordingly, it is possible to set the pulse non-application time period Toff more appropriately according to the thermal relaxation time period of collagen fibers.

In the present embodiment, the pulse non-application time period Toff is set equal to or more than the thermal relaxation time period of collagen fibers.

Accordingly, it is possible to assuredly inhibit a temperature rise of collagen blast cells and eventually a damage of fibroblast.

In the present embodiment, the pulse non-application time period Toff is between 80 [ms] and 210 [ms] inclusive.

As is clear from FIGS. 5 and 6, according to such a configuration, it is possible to set the pulse non-application time period Toff enabling the temperature of water to decrease according to the wavelength of light and the pulse application time period Ton that make it possible to obtain a preferable temperature rise ratio.

In the present embodiment, the pulse application time period Ton is set such that the temperature of water at the end of the pulse application time period Ton is equal to or lower than the second temperature (given temperature).

Accordingly, it is possible to more assuredly inhibit a temperature rise of fibroblast and eventually a damage of fibroblast.

In the present embodiment, the second temperature is a thermal denaturation threshold temperature at which collagen molecules in collagen fibers thermally denature.

Accordingly, it is possible to thermally denature collagen molecules.

In the present embodiment, the second temperature is a reversible thermal denaturation threshold temperature at which the collagen molecules in the collagen fibers reversibly thermally denature.

Accordingly, it is possible to reversibly thermally denature collagen molecules.

In the present embodiment, the wavelength and the pulse application time period Ton are set such that the collagen molecules in collagen fibers thermally denature and fibroblast are not thermally damaged.

Accordingly, it is possible to inhibit a thermal damage of fibroblast while thermally denaturing collagen molecules.

In the present embodiment, the wavelength and the pulse application time period Ton are set such that collagen molecules in collagen fibers thermally denature and fibroblast are reversibly damaged.

Accordingly, it is possible to inhibit a thermal damage of fibroblast while thermally denaturing collagen molecules.

In the present embodiment, the wavelength and the pulse application time period Ton are set such that collagen molecules in collagen fibers irreversibly thermally denature and fibroblast are not thermally damaged.

Accordingly, it is possible to inhibit a thermal damage of fibroblast while irreversibly thermally denaturing collagen molecules.

In the present embodiment, the wavelength and the pulse application time period Ton are set such that the collagen molecules in collagen fibers irreversibly thermally denature and fibroblast reversibly thermally denature.

Accordingly, it is possible to inhibit a thermal damage of fibroblast while irreversibly thermally denaturing collagen molecules.

In the present embodiment, the wavelength and the pulse application time period Ton are set such that thermal transpiration occurs on the surface of the skin S.

Accordingly, it is possible to give coagulation treatment in deep tissue.

In the present embodiment, the wavelength, the pulse application time period Ton, the pulse non-application time period Toff, and the number of times the pulse application time period Ton is repeated are set such that collagen molecules in collagen fibers thermally denature and fibroblast are not thermally damaged.

Accordingly, it is possible to inhibit a thermal damage of fibroblast while thermally denaturing collagen molecules.

In the present embodiment, the wavelength, the pulse application time period Ton, the pulse non-application time period Toff, and the number of times the pulse application time period Ton is repeated are set such that collagen molecules in collagen fibers thermally denature and fibroblast are reversibly thermally damaged.

Accordingly, it is possible to inhibit a thermal damage of fibroblast while thermally denaturing collagen molecules.

In the present embodiment, the wavelength, the pulse application time period Ton, the pulse non-application time period Toff, and the number of times the pulse application time period Ton is repeated are set such that collagen molecules in collagen fibers reversibly thermally denature and fibroblast are not thermally damaged.

Accordingly, it is possible to inhibit a thermal damage of fibroblast while irreversibly thermally denaturing collagen molecules.

In the present embodiment, the wavelength, the pulse application time period Ton, the pulse non-application time period Toff, and the number of times the pulse application time period Ton is repeated are set such that collagen molecules in collagen fibers reversibly thermally denature and fibroblast are reversibly damaged.

Accordingly, it is possible to inhibit a thermal damage of fibroblast while irreversibly thermally denaturing collagen molecules.

In the present embodiment, the wavelength, the pulse application time period Ton, the pulse non-application time period Toff, and the number of times the pulse application time period Ton is repeated are set such that thermal transpiration occurs on the surface of the skin S.

Accordingly, it is possible to remove shallow tissue and thus give coagulation treatment in deep tissue.

In the present embodiment, the ratio of the temperature rise width of the collagen fibers to the temperature rise width of water when light is applied to the skin S is equal to or larger than 1.1.

As is clear from FIG. 5, according to such a configuration, a higher temperature rise ratio is obtained. In other words, it is possible to selectively heat collagen fibers more efficiently.

In the present embodiment, at the time when the given denaturation state of collagen fibers is detected by the sensor 50 (detector), application of a pulse ends.

Accordingly, advantages that it is possible to inhibit excessive application of light and omit unnecessary application of light are obtained.

In the present embodiment, in the pulse application time period Ton, a plurality of pulses of light are applied intermittently.

Accordingly, the same effect as that in the case where laser light is applied successively in the pulse application time period Ton can be obtained.

In the present embodiment, the wavelength of laser light is set at a wavelength at which the absorption coefficient of collagen fibers is larger than the absorption coefficient of water.

Accordingly, it is possible to efficiently increase the temperature of collagen fibers while inhibiting a temperature rise of the fibroblast in the pulse application time period Ton.

In the present embodiment, the wavelength of light is set at a value longer than 1480 [nm].

Accordingly, it is possible to more efficiently increase the temperature of collagen fibers while inhibiting a temperature rise of the fibroblast in the pulse application time period Ton.

In the present embodiment, the wavelength of light is set equal to or less than 1600 [nm].

Accordingly, it is possible to more efficiently increase the temperature of collagen fibers while inhibiting a temperature rise of the fibroblast in the pulse application time period Ton.

The present embodiment of the present invention and modifications thereof have been exemplified and the present embodiment and the modifications described above are an example and are not intended to limit the scope of the invention. The above-described embodiment and modifications can be carried out in other various modes and various types of omission, replacement, combination and change can be made without departing from the scope of the invention. Specification, such as each configuration and the shape (the structure, type, direction, model, size, length, width, thickness, height, number, arrangement, position, and material) can be changed as appropriate and enabled.

For example, the living tissue is not limited to skin, and it may be, for example, living tissue containing collagen fibers, such as the alimentary tract, cartilage, or bone. Furthermore, cells may be cells other than fibroblast.

The pulse application number-of-times may be set according to the temperature of water or collagen fibers.

When the wavelength is set longer than 1600 [nm], because the absorption coefficient is relatively small and accordingly a light invasion length is relatively long, it is possible to obtain the same function and effect on a deeper part of living tissue.

Industrial Applicability

The present invention is usable in a pulse application method and a pulse application device.

Reference Signs List

1    PULSE APPLICATION DEVICE

10    PULSE LASER DEVICE
20    OPTICAL HEAD
30    OPTICAL FIBER
40    CONTROL DEVICE
50    SENSOR (DETECTOR)
S    SKIN
SI    LIVING ORGANISMS WATER
S2    COLLAGEN FIBER BUNDLE
S3    FIBROBLAST
Toff    PULSE NON-APPLICATION TIME PERIOD
Ton    PULSE APPLICATION TIME PERIOD

**Claims**

**1.** A pulse application method of applying a pulse of light to living tissue to heat the living tissue, wherein a wavelength of the light is set within a range in which a temperature rise width of collagen fibers in the living tissue when the light is applied to the living tissue is larger than a temperature rise width of water containing cells that are contained in the living tissue and that are present around the collagen fibers.

2. The pulse application method according to claim 1, wherein the cells are fibroblast.

3. The pulse application method according to claim 1, wherein an application time period in which a pulse of light is applied to the living tissue and a non-application time period in which no pulse of light is applied to the living tissue are repeated alternately.

4. The pulse application method according to claim 3, wherein the non-application time period is set such that a temperature of the water at an end of the non-application time period is equal to or lower than a first temperature.

5. The pulse application method according to claim 4, wherein the non-application time period is set such that the temperature of the water at the end of the non-application time period is approximately equal to the temperature of the water at a start of the application time period immediately before the non-application time period.

6. The pulse application method according to any one of claims 3 to 5, wherein the non-application time period is set such that a temperature of the collagen fibers at an end of the non-application time period is higher than the temperature of the collagen fibers at a start of the application time period immediately before the non-application time period.

7. The pulse application method according to any one of claims 3 to 6, wherein the non-application time period is set based on a thermal relaxation time period from an end of the application time period immediately before the non-application time period until a time when a temperature of the collagen fibers decreases to a temperature obtained by adding a temperature width obtained by dividing the temperature rise width of the collagen fibers in the application time period by a bottom of a natural logarithm to a temperature at a start of the application time period.

8. The pulse application method according to claim 7, wherein the non-application time period is set equal to or more than the thermal relaxation time period.

9. The pulse application method according to any one of claims 3 to 8, wherein the non-application time period is between 80 [ms] and 210 [ms] inclusive.

10. The pulse application method according to any one of claims 1 to 9, wherein the wavelength and an application time period of the pulse are set such that the temperature of the water at an end of the application time period is equal to or lower than a second temperature.

11. The pulse application method according to claim 10, wherein the second temperature is a thermal denaturation threshold temperature at which collagen molecules in collagen fibers thermally denature.

12. The pulse application method according to claim 10, wherein the second temperature is a reversible thermal denaturation threshold temperature at which collagen molecules in collagen fibers reversibly thermally denature.

13. The pulse application method according to claim 1, wherein the wavelength and an application time period of the pulse are set such that collagen molecules in the collagen fibers thermally denature and the cells are not thermally damaged.

14. The pulse application method according to claim 1, wherein the wavelength and an application time period of the pulse are set such that collagen molecules in the collagen fibers thermally denature and the cells are reversibly thermally damaged.

15. The pulse application method according to claim 1, wherein the wavelength and an application time period of the pulse are set such that collagen molecules in the collagen fibers irreversibly thermally denature and the cells are not thermally damaged.

16. The pulse application method according to claim 1, wherein the wavelength and an application time period of the pulse are set such that collagen molecules in the collagen fibers irreversibly thermally denature and the cells are reversibly thermally damaged.

17. The pulse application method according to any one of claims 1 and 13 to 16, wherein the wavelength and an application time period of the pulse are set such that thermal transpiration occurs on a surface of the living tissue.

18. The pulse application method according to claim 3, wherein the application time period in which the pulse of light is applied to the living tissue and the non-application time period in which no pulse of light is applied to the living tissue are repeated alternately, and
the wavelength, the application time period, the non-application time period, and the number of times the application time period is repeated are set such that collagen molecules in the collagen fibers thermally denature and the cells are not thermally damaged.

19. The pulse application method according to claim 3, wherein the application time period in which the pulse of light is applied to the living tissue and the non-application time period in which no pulse of light is applied to the living tissue are repeated alternately, and
the wavelength, the application time period, the non-application time period, and the number of times the application time period is repeated are set such that collagen molecules in the collagen fibers thermally denature and the cells are reversibly thermally damaged.

20. The pulse application method according to claim 3, wherein the application time period in which the pulse of light is applied to the living tissue and the non-application time period in which no pulse of light is applied to the living tissue are repeated alternately, and
the wavelength, the application time period, the non-application time period, and the number of times the application time period is repeated are set such that collagen molecules in the collagen fibers irreversibly thermally denature and the cells are not thermally damaged.

21. The pulse application method according to claim 3, wherein the application time period in which the pulse of light is applied to the living tissue and the non-application time period in which no pulse of light is applied to the living tissue are repeated alternately, and
the wavelength, the application time period, the non-application time period, and the number of times the application time period is repeated are set such that collagen molecules in the collagen fibers irreversibly thermally denature and the cells are reversibly thermally damaged.

22. The pulse application method according to any one of claims 3 and 18 to 21, wherein the application time period in which the pulse of light is applied to the living tissue and the non-application time period in which no pulse of light is applied to the living tissue are repeated alternately, and
the wavelength, the application time period, the non-application time period, and the number of times the application time period is repeated are set such that thermal transpiration occurs on a surface of the living tissue.

23. The pulse application method according to any one of claims 1 to 22, wherein a ratio of the temperature rise width of the collagen fibers to the temperature rise width of the water when the light is applied to the living tissue is equal to or larger than 1.1.

24. The pulse application method according to any one of claims 1 to 23, wherein

a denaturation state of the collagen fibers is detected, and
at a time when a given denaturation state of the collagen fibers is detected, application of the pulse ends.

25. The pulse application method according to any one of claims 1 to 24, wherein, in an application time period of the pulse, a plurality of pulses of light are applied intermittently.

26. The pulse application method according to any one of claims 1 to 25, wherein the wavelength of the light is set at a wavelength at which an absorption coefficient of the collagen fibers is larger than an absorption coefficient of the water.

27. The pulse application method according to any one of claims 1 to 26, wherein the wavelength of the light is set at a value longer than 1480 [nm].

**28.** The pulse application method according to any one of claims 1 to 27, wherein the wavelength of the light is set at a value equal to or less than 1600 [nm].

**29.** A pulse application device configured to apply a pulse of light to living tissue to heat the living tissue, wherein a wavelength of the light is set within a range in which a temperature rise width of collagen fibers in the living tissue when the light is applied to the living tissue is larger than a temperature rise width of water containing fibroblast that are contained in the living tissue and that are present around the collagen fibers.

**30.** The pulse application device according to claim 29, comprising a detector configured to detect a denaturation state of the collagen fibers,
wherein at a time when a given denaturation state of the collagen fibers is detected by the detector, application of the pulse ends.

**31.** The pulse application device according to claim 29 or 30, wherein the wavelength of the light is set at a value longer than 1480 [nm].

**32.** The pulse application device according to any one of claims 29 to 31, wherein the wavelength of the light is set at a value equal to or less than 1600 [nm].

# FIG.1

# FIG.2

# FIG.3

# FIG.4

# FIG.5

(Graph) Y-axis: TEMPERATURE RISE RATIO (0.80 to 1.70), X-axis: PULSE APPLICATION TIME PERIOD (Ton) [$\mu$s] (0.01 to 1000)

Legend:
- 1480nm
- 1500nm
- 1530nm
- 1550nm
- 1570nm
- 1600nm

# FIG.6

(Graph) Y-axis: PULSE NON-APPLICATION TIME PERIOD (Toff) [ms] (50 to 250), X-axis: PULSE APPLICATION TIME PERIOD (Ton) [$\mu$s] (0.01 to 1000)

Legend:
- 1480nm
- 1500nm
- 1530nm
- 1550nm
- 1570nm
- 1600nm

18

**INTERNATIONAL SEARCH REPORT**

| International application No. |
| --- |
| PCT/JP2020/043058 |

A.  CLASSIFICATION OF SUBJECT MATTER
Int. Cl. A61B18/20(2006.01)i, A61N5/067(2006.01)i
FI: A61B18/20, A61N5/067

According to International Patent Classification (IPC) or to both national classification and IPC

B.  FIELDS SEARCHED

Minimum documentation searched (classification system followed by classification symbols)
Int. Cl. A61B18/20, A61N5/067

Documentation searched other than minimum documentation to the extent that such documents are included in the fields searched
Published examined utility model applications of Japan      1922-1996
Published unexamined utility model applications of Japan    1971-2020
Registered utility model specifications of Japan            1996-2020
Published registered utility model applications of Japan    1994-2020

Electronic data base consulted during the international search (name of data base and, where practicable, search terms used)

C.  DOCUMENTS CONSIDERED TO BE RELEVANT

| Category* | Citation of document, with indication, where appropriate, of the relevant passages | Relevant to claim No. |
| --- | --- | --- |
| X | US 2008/0269733 A1 (ANDERSON, R. Rox) 30 October 2008, fig. 1-4, paragraphs [0007], [0020], [0021], [0031]-[0034] | 1-3, 6, 10-23, 26-29, 31-32 |
| Y |  | 4-5, 7-9, 24-25, 30 |
| Y | WO 2018/119453 A1 (THE GENERAL HOSPITAL CORPORATION) 28 June 2018, paragraphs [0010], [0011], [0111], [0112] | 4-5, 7-8 |
| Y | JP 4-506312 A (SAND, BRUCE J) 05 November 1992, page 7, lower right column, lines 1-21 | 9, 25 |
| Y | JP 2002-507927 A (BRONCUS TECHNOLOGIES, INC.) 12 March 2002, page 17, lines 22-27 | 24, 30 |

☒  Further documents are listed in the continuation of Box C.    ☒  See patent family annex.

| * | Special categories of cited documents: | "T" | later document published after the international filing date or priority date and not in conflict with the application but cited to understand the principle or theory underlying the invention |
| --- | --- | --- | --- |
| "A" | document defining the general state of the art which is not considered to be of particular relevance | | |
| "E" | earlier application or patent but published on or after the international filing date | "X" | document of particular relevance; the claimed invention cannot be considered novel or cannot be considered to involve an inventive step when the document is taken alone |
| "L" | document which may throw doubts on priority claim(s) or which is cited to establish the publication date of another citation or other special reason (as specified) | "Y" | document of particular relevance; the claimed invention cannot be considered to involve an inventive step when the document is combined with one or more other such documents, such combination being obvious to a person skilled in the art |
| "O" | document referring to an oral disclosure, use, exhibition or other means | | |
| "P" | document published prior to the international filing date but later than the priority date claimed | "&" | document member of the same patent family |

| Date of the actual completion of the international search | Date of mailing of the international search report |
| --- | --- |
| 15.12.2020 | 22.12.2020 |

| Name and mailing address of the ISA/ | Authorized officer |
| --- | --- |
| Japan Patent Office 3-4-3, Kasumigaseki, Chiyoda-ku, Tokyo 100-8915, Japan | |
| | Telephone No. |

Form PCT/ISA/210 (second sheet) (January 2015)

**INTERNATIONAL SEARCH REPORT**

International application No.

PCT/JP2020/043058

C (Continuation).   DOCUMENTS CONSIDERED TO BE RELEVANT

| Category* | Citation of document, with indication, where appropriate, of the relevant passages | Relevant to claim No. |
|---|---|---|
| A | US 2013/0245616 A1 (TOYOTA JIDOSHA KABUSHIKI KAISHA) 19 September 2013, entire text | 1-32 |

Form PCT/ISA/210 (continuation of second sheet) (January 2015)

| | International application No. |
|---|---|
| | PCT/JP2020/043058 |

| Patent Documents referred to in the Report | Publication Date | Patent Family | Publication Date |
|---|---|---|---|
| US 2008/0269733 A1 | 30.10.2008 | WO 1998/033558 A1 page 3, lines 6-11, page 5, line 17 to page 6, line 2, page 8, line 17 to page 9, line 17, fig. 1-4 EP 1011811 A1 | |
| WO 2018/119453 A1 | 28.06.2018 | JP 2020-503108 A paragraphs [0010], [0011], [0091], [0092] US 2018/0177550 A1 CN 110800081 A | |
| JP 4-506312 A | 05.11.1992 | US 4976709 A column 8, lines 28-54 WO 1995/028135 A1 | |
| JP 2002-507927 A | 12.03.2002 | US 5957919 A column 5, lines 37-47 WO 99/01076 A1 | |
| US 2013/0245616 A1 | 19.09.2013 | WO 2011/133660 A2 entire text | |

Form PCT/ISA/210 (patent family annex) (January 2015)

21

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Non-patent literature cited in the description**

- **JASON PREISSIG ; KRISTY HAMILTON ; RAMSEY MARKUS.** Current Laser Resurfacing Technologies: A Review that Delves Beneath the Surface. *Seminars in Plastic Surgery,* 2012, vol. 26 (3), 109-116
- **BANRI ONO.** *The Journal of Japan Society for Laser Surgery and Medicine,* 2015, vol. 36, 324
- **KOU et al.** *Appl Opt,* 1993, vol. 32, 3521-3540
- **MINORU OBARA ; TSUNENORI ARAI ; KATSUMI MIDORIKAWA.** Applied Laser Engineering. Corona Publishing, 1998
- **RR ANDERSON ; JA PARRISH.** Selective photothermolysis: precise microsurgery by selective absorption of pulsed radiation. *Science,* 1983, vol. 220 (4596), 524-527
- **NEIL T. WRIGHT.** On a Relationship Between the Arrhenius Parameters from Thermal Damage Studies. *Journal of Biomechanical Engineering,* 2003, vol. 125 (2), 300-304
- **T. N. GLENN ; S. RASTEGAR ; S. L. JACQUES.** Finite element analysis of temperature controlled coagulation in laser irradiated tissue. *IEEE Transactions on Biomedical Engineering,* January 1996, vol. 43 (1), 79-87
- **JEAN-LUC BOULNOIS.** Photophysical processes in recent medical laser developments: A review. *Lasers in Medical Science,* January 1986, vol. 1 (1), 47-66